(19) Europäisches Patentamt
European Patent Office
Office européen des brevets

(11) EP 0 629 134 B1

# (12) EUROPEAN PATENT SPECIFICATION

(45) Date of publication and mention of the grant of the patent:
**17.05.2000 Bulletin 2000/20**

(21) Application number: **93907259.1**

(22) Date of filing: **04.03.1993**

(51) Int. Cl.[7]: **A61L 2/00**, A01N 59/00

(86) International application number:
**PCT/US93/02018**

(87) International publication number:
**WO 93/17721 (16.09.1993 Gazette 1993/22)**

## (54) USE OF ASPERGILLUS NIGER CATALASE-R FOR HYDROGEN PEROXIDE NEUTRALIZATION

VERWENDUNG VON ASPERGILLUS NIGER CATALASE-R ZUR WASSERSTOFFPEROXID-NEUTRALISIERUNG

NEUTRALISATION DE L'EAU OXYGENEE PAR LA CATALASE-R ASPERGILLUS NIGER

(84) Designated Contracting States:
**AT BE CH DE DK ES FR GB GR IE IT LI LU MC NL PT SE**

(30) Priority: **04.03.1992 US 845990**

(43) Date of publication of application:
**21.12.1994 Bulletin 1994/51**

(60) Divisional application:
**99119007.5 / 0 995 796**

(73) Proprietor:
**GENENCOR INTERNATIONAL, INC.
Rochester, New York 14618 (US)**

(72) Inventors:
- **BERKA, Randy M.
Davis, CA 95616 (US)**
- **FOWLER, Timothy
Redwood City, CA 94061 (US)**
- **VAHA-VAHE, Pekka
SF-02460 Kantvik (FI)**

(74) Representative:
**Brandes, Jürgen, Dr. et al
Wuesthoff & Wuesthoff
Patent- und Rechtsanwälte
Schweigerstrasse 2
81541 München (DE)**

(56) References cited:
- **No relevant documents disclosed**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

Field of the Invention:

The invention relates to the application of an *Aspergillus niger* catalase enzyme for the neutralization of hydrogen peroxide in solution. It was discovered that *A. niger* produces two catalases, designated as catalase-A and catalase-R. When compared to catalase-A and beef liver catalase, catalase-R shows superior performance for biotechnological applications. Furthermore, the *A. niger* catalase-R enzyme is shown to be approximately four-times as effective as beef liver catalase in applications requiring the neutralization of concentrated hydrogen peroxide solutions.

Background of the Invention:

Catalases [hydrogen peroxide: hydrogen peroxide oxidoreductases (EC 1.11.1.6)] are enzymes which catalyze the conversion of hydrogen peroxide ($H_2O_2$) to oxygen ($O_2$) and water ($H_2O$) according to the following formula:

$$2H_2O_2 \xrightarrow{\text{Catalase}} 2H_2O + O_2$$

These ubiquitous enzymes have been purified from a variety of animal tissues, plants and microorganisms (Chance and Maehly 1955 Methods Enzymol. **2**: 764-791; Jones and Wilson 1978 in H. Sigel (ed.), *Metal Ions in Biological Systems*, Vol. 7, Marcel Dekker Inc., New York). Nearly all forms of the enzyme which have been characterized consist of four polypeptide subunits, each having a molecular weight of 50,000 to 60,000 and containing one protohemin prosthetic group per subunit (Wasserman and Hultin 1981 Arch. Biochem. Biophys. **212**: 385-392; Hartig and Ruis 1986 Eur. J. Biochem. **160**: 487-490).

Bovine liver catalase has been the most extensively studied variety of this enzyme [Schonbaum and Chance 1976 in *The Enzymes* (P.D. Boyer, ed.) 3rd edn., vol. 13, pp. 363-408, Academic Press, New York]. The complete amino acid sequence and three dimensional structure of bovine liver catalase are known (Schroeder, et al., 1982 Arch. Biochem. Biophys. **214**: 397-412; Murthy, et al., 1981 J. Mol. Biol. **152**: 465-499).

Although less well-studied from a biochemical and biophysical standpoint, catalases from filamentous fungi have several characteristics that distinguish them from their mammalian counterparts. While similar in subunit number and heme content, fungal catalases are substantially larger molecules than those from other organisms, having subunit molecular weights ranging from 80,000 to 97,000 (Vainshtein, et al., 1986 J. Mol. Biol. **188**: 63-72; Jacob and Orme-Johnson 1979 Biochem. **18**: 2967-2975; Jones, et al., 1987 Biochim. Biophys. Acta **913**: 395-398). More importantly, catalases from fungi such as *Aspergillus niger* are more stable than beef liver catalase to proteolysis and to inactivation by glutaraldehyde, SDS, and have lower affinity for catalase inhibitors such as cyanide, azide and fluoride (Wasserman and Hultin 1981 Arch. Biochem. Biophys. **212**: 385-392). In addition, *A. niger* catalase is significantly more stable than bovine liver catalase when subjected to extremes of pH, hydrogen peroxide, and temperature (Scott and Hammer 1960 Enzymolagia **22**: 229-237). Although fungal catalases offer stability advantages, the corresponding mammalian enzymes such as beef liver catalase generally have higher catalytic activity (Gruft, et al., 1978; Can. J. Biochem. **56**: 916-919). Traditionally, beef liver catalase has been the preferred enzyme for diagnostic purposes and for pharmaceutical-related applications (e.g., contact-lens cleaning/disinfection/$H_2O_2$ neutralization). However, recent outbreaks of a slow-virus disease known as BSE (bovine spongiform encephalopathy) in European cattle herds and fear that this disease might be spread to man [Dealler and Lacey 1991 Nutr. Health (Bicester) **7**: 117-134; Dealler and Lacey 1990 Food Microbiol. **7**: 253-280] have aroused interests in finding alternatives to beef liver catalase for these applications. Furthermore, since enzyme stability is an important factor in the biotechnological utilization of enzymes, there is considerable interest in the use of *A. niger* catalase, in applications involving neutralization of high concentrations of hydrogen peroxide and for pharmaceutical-related applications in which the use of bovine liver catalase could be a health risk.

Although there are a number of published reports describing the biochemical and biophysical properties of *A. niger* catalase, it has not been previously disclosed that there are in fact two catalase enzymes present in this organism. Using standard molecular biology techniques, we have isolated genes (*catA* and *catR*) encoding two different catalase enzymes (catalase-A and catalase-R) from *A. niger* (Genencor International, Inc., unpublished). The *A. niger catA* gene, cloned by cross-hybridization to the yeast (*Saccharomyces cerevisiae*) *CTA1* gene, encodes a catalase enzyme which is induced primarily during growth on fatty acids and is presumably peroxisomal. *A. niger* catalase-A is less stable (i.e., has a shorter half-life in storage), is inactivated more rapidly by high concentrations of hydrogen peroxide, and less active than catalase-R at low pH. The *A. niger catR* gene encodes a soluble cytoplasmic enzyme (catalase-R) which we discovered to be the major activity in commercial catalase preparations. In contrast to catalase-A, this enzyme is stable over a wide pH range, has an extended shelf life, and is resistant to deactivation in high concentrations of hydrogen peroxide. In addition, we have discovered that catalase-R is more effective than beef liver catalase for neutralization of concentrated (e.g., three percent) hydrogen peroxide solutions.

Summary of the Invention:

It has been discovered that the *A. niger* genome contains two catalase genes, designated *catA* and *catR*. In addition, it was discovered that the *catA* and *catR* gene products (catalase-A and catalase-R, respectively) are two different catalase enzymes, each having their own distinctive characteristics. The invention discloses that catalase-R has characteristics which make it a superior enzyme for a number of biotechnological applications involving neutralization of hydrogen peroxide solutions. In addition, the invention includes the discovery that catalase-R shows performance which is superior to beef liver catalase in removal of hydrogen peroxide.

Accordingly, the present invention relates to a catalase-R enzyme comprising the amino acid sequence given in Figure 5A-C or allelic variations thereof. In addition, the invention relates to a DNA molecule coding for the above mentioned catalase-R enzyme. Finally, the subject matter of the present invention is a method for neutralisation of hydrogene peroxide in a solution comprising treating said solution with an effective amount of the catalase-R enzyme. Finally, the present invention concerns a method of cleaning and disinfecting contact lenses comprising treating the lens with a solution of hydrogen peroxide having a strength sufficient to disinfect the lens, and decomposing the residual hydrogen peroxide in the solution by exposing said solution to an amount of the catalase-R enzyme which is effective to decompose the residual hydrogen peroxide.

Figures:

Figure 1 displays a comparison of catalase-A and catalase-R activities in dilute hydrogen peroxide solution (100 ppm).

Figure 2 shows a comparison of catalase-A and catalase-R activities in concentrated hydrogen peroxide solution (16.5 g/L).

Figure 3 illustrates a comparison of enzymatic activities of catalase-A and catalase-R at different pH values.

Figure 4 shows restriction maps of the *A. niger catA* and *catR* genes encoding catalase-A and catalase-R, respectively.

Figure 5 displays the nucleotide sequence and deduced amino acid sequence of the *A. niger catR* gene. Introns are denoted by dashed lines.

Figure 6 shows a comparison of the effectiveness of catalase-R and beef liver catalase tablets (each containing 1 mg of enzyme) at neutralizing a 3% solution of hydrogen peroxide. Specific activities for the two enzyme preparations were comparable (6541 U/mg for beef liver catalase, and 7344 U/mg for *A. niger* catalase-R).

Description of the Preferred Embodiments.

A comparison of the biochemical and biophysical properties of catalase-A and catalase-R is described below. Additionally, a comparison of catalase-R and beef liver catalase enzyme performance is outlined. One skilled in the art will understand that various changes in the following examples could be made. Accordingly, the examples are not intended to be limiting.

1. Comparison of the biochemical and biophysical properties of catalase-A and catalase-R.

Techniques used to measure biochemical parameters such as enzyme activity, hydrogen peroxide concentrations, pH-activity profile, and enzyme stability are conventional techniques described in the following examples.

*Purification of A. niger catalase-R*

Catalase-R was purified from a commercial preparation of *A. niger* catalase (Fermcolase 1000, Genencor International, Inc.) using ion exchange chromatography on TrisAcryl Q resin (IBF Biotechnics, Inc.). First, the commercial enzyme preparation was desalted on a PD-10 column (Pharmacia-LKB) equilibrated in 10 mM Tris-HC1 at pH 8. The enzyme was loaded onto a 2.5 cm x 5.5 cm TrisAcryl Q column equilibrated first with 100 ml of 100 mM Tris-HC1, pH 8, then with 200 ml of 10 mM Tris-HC1, pH 8 at a flow rate of 6.0 ml/min. Unbound proteins were eluted by washing the column with 10 mM Tris-HC1. The remaining proteins were eluted with a gradient of increasing NaC1 (zero to 0.5 M in 10 mM Tris-HC1, pH 8). The protein fraction having the highest $A_{410}/A_{280}$ ratio contained purified catalase. SDS-PAGE analysis of this protein fraction showed a single-band which migrated with an apparent molecular weight of approximately 80,000.

*Purification of A. niger catalase-A*

*A. niger* catalase-A was purified from mycelial extracts of cells grown for two days in medium which contained 1% glucose as the carbon source. The mycelia were quick-frozen in liquid nitrogen and ground to a fine powder in an electric coffee-grinder. The frozen powder was suspended in 10 ml of 100 mM sodium formate buffer, pH 7 and kept on ice for one hour. The insoluble debris was removed by centrifugation, and the supernatant was concentrated by ultrafiltration (Amicon). The concentrated extract was then chromatographed on Sephacryl S-100 and the fractions with catalase activity were rechromatographed on an ion exchange column as described above. Two peaks, one major and one minor, containing catalase activity were analyzed by SDS-PAGE. The major catalase fraction gave a predominant band with a molecular weight of 80,000 and was subsequently shown to be catalase-R. The minor component, catalase-A, gave a band with molecular weight of 46,000.

Table 1 shows a comparison of the biochemical and biophysical properties of *A. niger* catalase-A and catalase-R.

Table 1

| Properties | Catalase-R | Catalase-A |
|---|---|---|
| Molecular weight | 80,000 | 46,000 |
| Specific activity (IU/mg) | 7500 | 6300 |
| Synthesis induced by | glucose | fatty acids |
| Cellular location | cytoplasmic | peroxisomal |
| Stability to high $H_2O_2$ | yes | no |
| Storage stability (35°C, 3 days, pH 7) | 55% | >90% |

2. <u>Cloning of the *A. niger catA* and *catR* genes.</u>

The techniques used in cloning the *A. niger catA* and *catR* genes are conventional techniques described in Sambrook, et al., 1989 *Molecular Cloning, A Laboratory Manual*, Cold Spring Harbor Press, Cold Spring Harbor, NY.

*Cloning of the A. niger catR gene*

A series of proteolytic fragments were generated from purified catalase-R by digestion of the protein with cyanogen bromide. These peptide fragments were subjected to amino acid sequence analysis. The amino acid sequence information was employed to design synthetic DNA probes for identification of *catR*-specific cDNA sequences in a λgt11 library. Briefly, the peptide fragment Met-Phe-Trp-Asn-Ser-Leu-Ile-Pro-Ala-Glu-Gln-Gln-Met was used to design a pool of three synthetic oligonucleotides having the following sequences:

```
5' ATG TTC TGG AAC AGC CTG ATC CCC GCC GAG CAG ATG 3'
5' ATG TTC TGG AAC TCC CTG ATC CCC GCC GAG CAG ATG 3'
5' ATG TTC TGG AAC AGC TTG ATC CCC GCC GAG CAG ATG 3'
```

This peptide was chosen because the amino acids give minimally degenerate codon choices. The differences among the three synthetic oligonucleotides represent alternate codon choices where there was no strong bias in the known codon usage pattern for *A. niger*. This position of this proteolytic fragment corresponds to peptide 3 shown in Figure 2. A clone containing a partial cDNA fragment was positively identified by hybridization with the synthetic DNA probe and nucleotide sequence analysis of this clone confirmed that it encoded catalase-R. This cloned cDNA segment was used to probe a library of *A. niger* genomic DNA. Subsequently, the entire *catR* gene, plus upstream and downstream transcriptional control elements, was assembled as a 9.0 kb *Hind*III - *Xho*I restriction fragment. The nucleotide sequence of the *catR* coding region has been determined and is given in Figure 5.

*Cloning of the A. niger catA gene*

The *A. niger catA* gene was cloned by cross-hybridization using the *S. cerevisiae CTA1* gene as the probe. The yeast catalase genes have been previously isolated and their sequences published (Cohen, et al., 1988 Eur. J. Biochem. **176**: 159-163; Hartig and Ruiz 1986, Eur. J. Biochem. **160**: 487-490). A fragment of the yeast *CTA1* gene was amplified using PCR (Perkin Elmer-Cetus) and cloned into pUC18. Sequence analysis of this clone confirmed it to be the yeast peroxisomal catalase gene. The clone was then used to screen Southern blots of *A. niger* FS-1 genomic DNA and found to hybridize to a single EcoRI band of 3.2 kb. The probe was then used to screen a λEMBL library of FS-1 genomic DNA, resulting in the identification of several positive clones, each containing the previously identified 3.2 kb *Eco*RI restriction fragment. One of these λEMBL clones (λEMBL3-1) was digested with *Bam*HI and a 9 kb fragment was subcloned into pUC18. A restriction map of this subclone is given in Figure 4. DNA sequence analysis of a portion of this fragment which hybridized with the yeast *CTA1* gene showed that the clone encoded a catalase that was different than catalase-R. On the basis of its homology with the yeast *CTA1* gene, it was given the designation *catA*. Co-transformations of *A. niger* FS-1 were done using the pUC18-*catA* plasmid and a plasmid containing a benomyl-resistance conferring β-tubulin gene. Benomyl-resistant colonies were screened for increased number of integrated *catA* gene copies by Southern blotting. Those transformants which showed more than one *catA* gene copy were evaluated in shake flask cultures. One transformant, designated 208, showed an approximate doubling in total cellular catalase activity.

3. <u>Performance comparison of *A. niger* catalase-R and beef liver catalase.</u>

The performance of purified catalase-R and purified beef liver catalase (Boehringer Mannheim) were compared in the following assays:

(a) Various amounts of each lyophilized enzyme powder were added to 10 ml of a 3% hydrogen peroxide solution as shown in Table 2. Specific activities of the two enzyme preparations were comparable (6541 U/mg for beef liver catalase, and 7344 U/mg for *A. niger* catalase-R). After 3 minutes, residual hydrogen peroxide was measure spectrophotometrically (Scott and Hammer 1960 Enzymologia **22**: 194-200). The results in Table 2 indicate that one-fourth the amount of catalase-R is required to neutralize 3% hydrogen peroxide relative to the amount of beef liver catalase needed. For example, 0.1 mg of catalase-R is equal in performance to 0.5 mg of beef liver catalase.

Table 2 discloses a comparison of the effectiveness of catalase-R and beef liver catalase at neutralizing a 3% solution of hydrogen peroxide in a 3 minute reaction. Specific activities of the two enzyme preparations were comparable (6541 U/mg for beef liver catalase, and 7344 U/mg for *A. niger* catalase-R).

Table 2

| Enzyme | Amt. of Enzyme per 10 ml | Peroxide remaining (ppm) |
|---|---|---|
| Beef liver | 1 mg | <1.4 |
| | 0.5 mg | <1.4 |
| | 0.2 mg | 109 |
| | 0.1 mg | >200 |
| | 0.05 mg | >200 |

Table 2 (continued)

| Enzyme | Amt. of Enzyme per 10 ml | Peroxide remaining (ppm) |
|---|---|---|
| Catalase-R | 1 mg | <1.4 |
| | 0.5 mg | <1.4 |
| | 0.2 mg | <1.4 |
| | 0.1 mg | <1.4 |
| | 0.05 mg | 125 |

(b) Tablets containing 1 mg of beef liver catalase were purchased in an Oxysept 2 kit (Allergan). Tablets containing 1 mg of *A. niger* catalase-R were prepared using sorbitol as the carrier. A comparison of the effectiveness of each of these tablets was done in a performance assay identical to that described above. Figure 6 shows that *A. niger* catalase-R tablets reduce 3% hydrogen peroxide to less than 1 ppm in 2 minutes, whereas, tablets containing beef liver catalase require 4 to 5 minutes.

## Claims

1. A catalase-R enzyme comprising the amino acid sequence given in Figure 5A-C or allelic variations thereof.

2. A DNA molecule coding for the catalase-R enzyme according to claim 1.

3. The DNA molecule according to claim 2 wherein said DNA sequence comprises the nucleotide sequence given in Figure 5A-C or allelic variations thereof.

4. A method for neutralization of hydrogen peroxide in a solution comprising treating said solution with an effective amount of the catalase-R enzyme according to claim 1.

5. A method of cleaning and disinfecting contact lens comprising treating the lens with a solution of hydrogen peroxide having a strength sufficient to disinfect the lens, and decomposing the residual hydrogen peroxide in the solution by exposing said solution to an amount of the catalase-R enzyme according to claim 1 which is effective to decompose the residual hydrogen peroxide.

6. The method according to claim 5 wherein said catalase-R is prepared with a carrier.

7. The method according to claim 6 wherein said carrier is sorbitol.

## Patentansprüche

1. Katalase-R-Enzym, umfassend die in Figur 5A-C angebene Aminosäuresequenz oder allelische Varianten davon.

2. DNA-Molekül, das für das Katalase-R-Enzym gemäß Anspruch 1 codiert.

3. DNA-Molekül nach Anspruch 2, wobei die DNA-Sequenz die in Figur FA-C angegebene Nukleotid-Sequenz aufweist oder allelische Varianten davon.

4. Verfahren zur Neutralisierung von Wasserstoffperoxid in einer Lösung, umfassend die Behandlung dieser Lösung mit einer wirksamen Menge eines Katalase-R-Enzyms nach Anspruch 1.

5. Verfahren zur Reinigung und Desinfizierung einer Kontaktlinse, umfassend das Behandeln der Kontaktlinse mit einer Wasserstoffperoxid-Lösung mit einer ausreichenden Stärke, um die Linse zu desinfizieren, und Zersetzung des restlichen Wasserstoffperoxids in der Lösung dadurch, daß man die Lösung einer Menge des Katalase-R-Enzyms nach Anspruch 1 aussetzt, die wirksam das restliche Wasserstoffperoxid zersetzt.

6. Verfahren nach Anspruch 5, wobei die Katalase-R mit einem Träger hergestellt wurde.

**7.** Verfahren nach Anspruch 6, wobei der Träger Sorbitol ist.

**Revendications**

**1.** Une enzyme catalase-R comprenant la séquence d'acides aminés donnée à la figure 5A-C ou des variations alléliques de celle-ci.

**2.** Une molécule d'ADN codant pour l'enzyme catalase-R selon la revendication 1.

**3.** La molécule d'ADN selon la revendication 2 dans laquelle ladite séquence d'ADN comprend la séquence nucléotidique donnée à la figure 5A-C ou des variations alléliques de celle-ci.

**4.** Un procédé pour la neutralisation du peroxyde d'hydrogène dans une solution, comprenant le traitement de ladite solution avec une quantité efficace de l'enzyme catalase-R selon la revendication 1.

**5.** Un procédé de nettoyage et de désinfection d'une lentille de contact comprenant le traitement de la lentille avec une solution de peroxyde d'hydrogène ayant une force suffisante pour désinfecter la lentille et la décomposition du peroxyde d'hydrogène résiduel dans la solution par l'exposition de ladite solution à une quantité de l'enzyme catalase-R selon la revendication I qui est efficace pour décomposer le peroxyde d'hydrogène résiduel.

**6.** Le procédé selon la revendication 5 dans lequel ladite catalase-R est préparée avec un véhicule.

**7.** Le procédé selon la revendication 6 dans lequel ledit véhicule est le sorbitol.

Figure 1

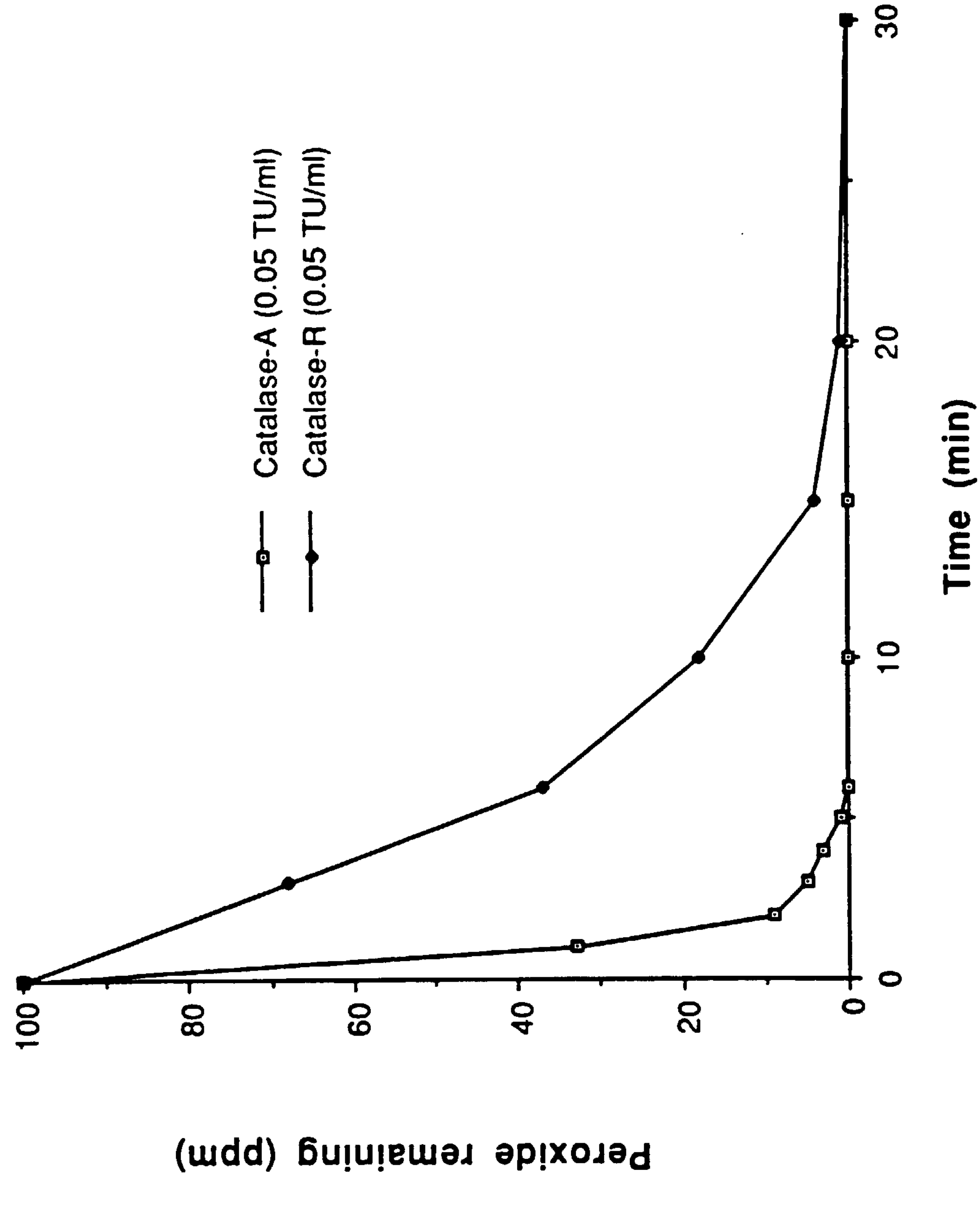
Catalase-A (0.05 TU/ml)
Catalase-R (0.05 TU/ml)
Peroxide remaining (ppm)
100
80
60
40
20
0
0
10
20
30
Time (min)

**Figure 2**

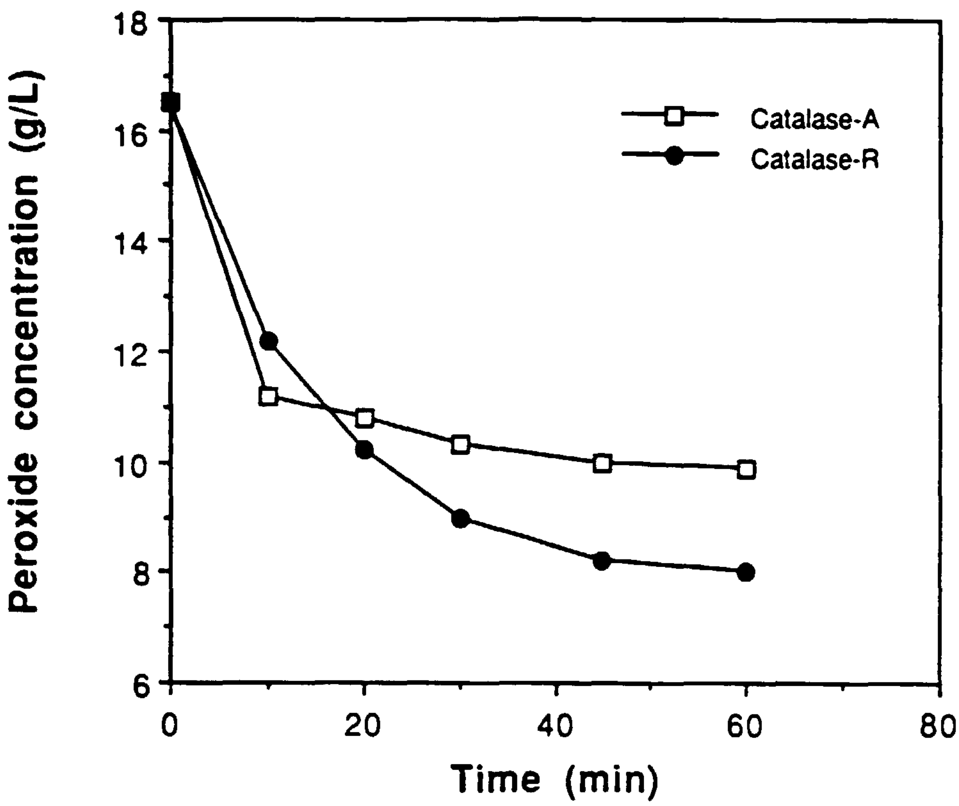
Peroxide concentration (g/L)
18
16
14
12
10
8
6
0
20
40
60
80
Time (min)
Catalase-A
Catalase-R

Figure 3

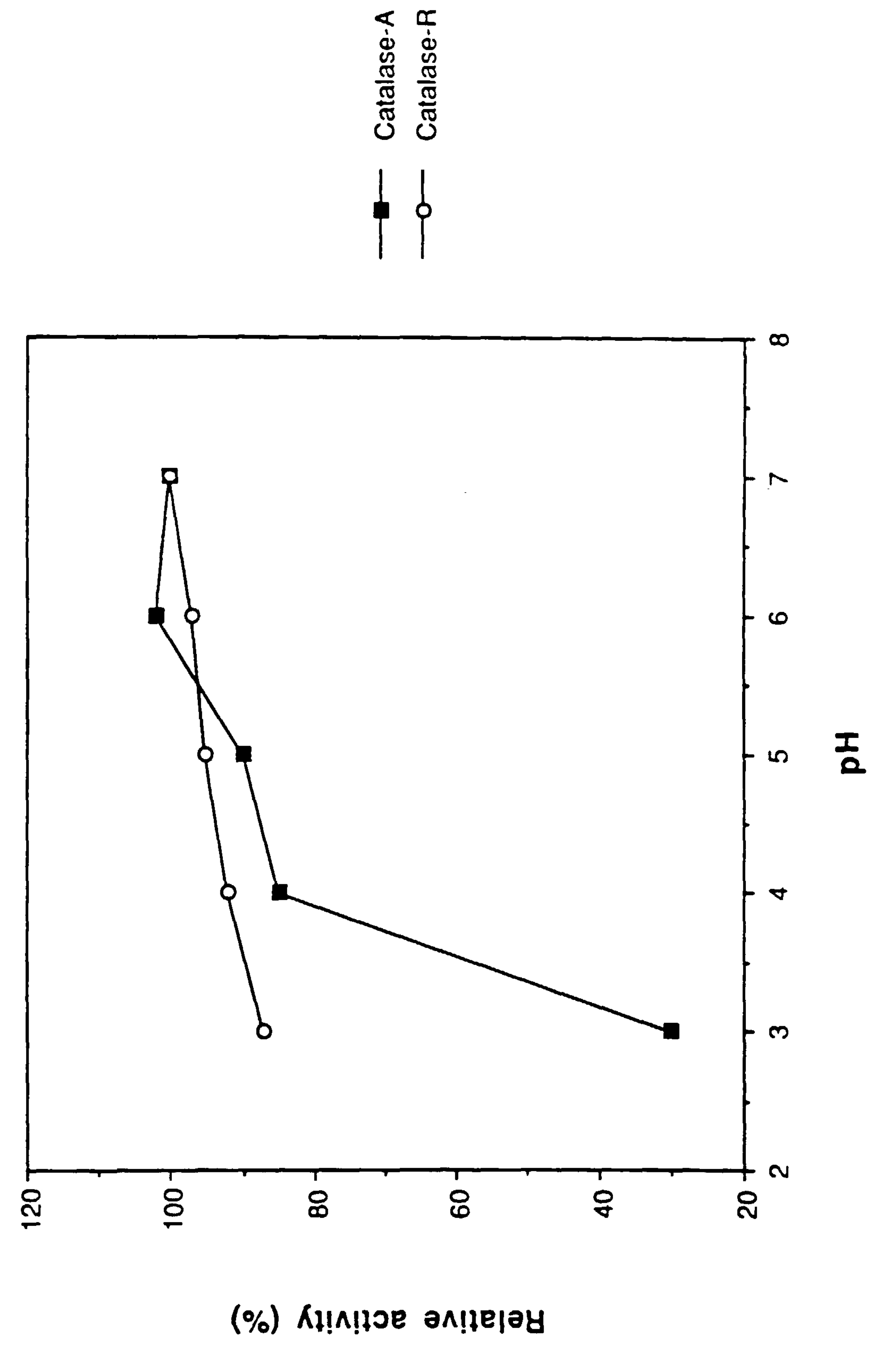
Catalase-A
Catalase-R
Relative activity (%)
120
100
80
60
40
20
pH
2
3
4
5
6
7
8

Figure 4

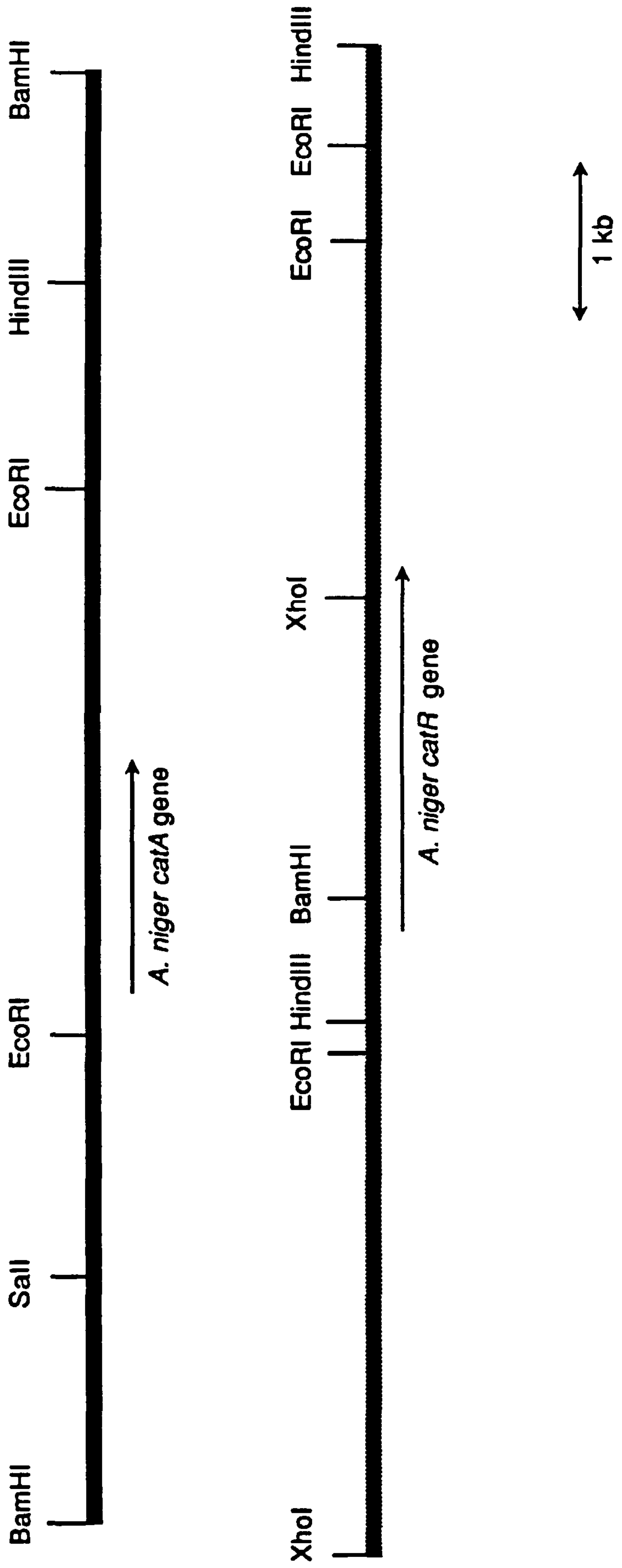
BamHI
SalI
EcoRI
EcoRI
HindIII
BamHI
*A. niger catA* gene
XhoI
EcoRI
HindIII
BamHI
XhoI
EcoRI
EcoRI
HindIII
*A. niger catR* gene
1 kb

```
CTTGTCACCGAGTGCCCGTTTGTCACTTGTTGTGGTGATCTTGAGCACATCGCGTTCCTCTCGTCTCATCACATCGAGTGATCAACATTG   90
CATGACCCTAGTGGAGCCCCTTCGTCTCCCAACAGGAGGGTCCGGATTACCAAGTCCCGACACCGTTTGGCTGTAATTCGACTCAAATTC  180
TGGATTCGTAGCTTAACTAAGACGCGTGGTCTGTTAACCGGCCTCGCCATGGATGCCGATATAAGGACCCTAGGGGACTCCCCCCTGGTG  270
ACTCTCGTCGGAAGATCGCAGCACTCTGAATTCTCCTAGTCTTCGTTTACTCCGCCATGCGTCATTTCTGCCTTTTGCCAGCTGTTGCTG  360
                                                       Met Arg His Phe Trp Leu Leu Pro Ala Val Ala
GTATCGCTGGGGCTCAATGCCCCTACCTGTCGGGTGAAATGAGTTTCACCCAGGAGCAGGACAATGCTGGCGATACCATTGAGGTCACGG  450
Gly Ile Ala Gly Ala Gln Cys Pro Tyr Leu Ser Gly Glu Met Ser Phe Thr Gln Glu Gln Asp Asn Ala Gly Asp Thr Ile Glu Val Thr
AGCAGCCCATTGACAACACCCTGTATGTCAATGACACCGGTAGCTACATGACTACCGACTTTGGCACTCCGATCTCCGACCAGACCAGTC  540
Glu Gln Pro Ile Asp Asn Thr Leu Tyr Val Asn Asp Thr Gly Ser Tyr Met Thr Thr Asp Phe Gly Thr Pro Ile Ser Asp Gln Thr Ser
TCAAGGCCGGGCCCCGTGGTCCTACCCTGTTGGAGGACTTTATCTTCCGTCAGAAGCTTCAGCGGTTCGACCATGAGCGTGTAAGTACAG  630
Leu Lys Ala Gly Pro Arg Gly Pro Thr Leu Leu Glu Asp Phe Ile Phe Arg Gln Lys Leu Gln Arg Phe Asp His Glu Arg-------
TAACTGCTGCGGTGTGTAGTAACAATAAATTGACCCAGTGGTTTTCAATTAGGTCCCCGAGCGCGTCGTCCACGCCCGTGGTGCCGGTGC  720
-----------------------------------------Val Pro Glu Arg Val Val His Ala Arg Gly   Gly Ala
ATATGGTACTTTCAAATCCTACGCCGACTGGTCGAACGTCACGGCTGCCGATTTCTTGAGTGCCAACGATAAGGAGAGCCCCTATGTTCTG  810
 Tyr Gly Thr Phe Lys Ser Tyr Ala Asp Trp Ser Asn Val Thr Ala Ala Asp Phe Leu Ser Ala Asn Asp Lys Glu Thr Pro Met Phe Cys
TCGCTTCTCTACTGTGGTCGGTTTCCGTGGTAGTGTTGACACTGCGCGTGATGTTCACGGTCACGCTTGTCGGTTCTACACTGACGAGGG  900
 Arg Phe Ser Thr Val Val Gly Phe Arg Gly Ser Val Asp Thr Ala Arg Asp Val His Gly His Ala Cys Arg Phe Tyr Thr Asp Glu Gly
TAACTATGGTATCTTGATATGGTCACCCAACAATAATTCAATACATGCTAACAGATATGTCTCTACTAGACATCGTCGGTATCAATTTCG  990
 Asn Tyr---------------------------------------------Asp Ile Val Gly Ile Asn Phe
CCCCCTTCTTCATCCAGGACGCCATCCAGTTCCCCGATCTTGTCCACGCCATCAAGCCCATGCCCAACAATGAGATCCCCCAGGCCGCTA  1080
Ala Pro Phe Phe Ile Gln Asp Ala Ile Gln Phe Pro Asp Leu Val His Ala Ile Lys Pro Met Pro Asn Asn Glu Ile Pro Gln Ala Ala
```

FIG. 5A

CTGCACACACTTCCGCTTGGGACTTCTTCAGCCAGCAGAGCACTGCCCTCCACAGTGCCTTGTGGCTGATGTCTGGTAACGGTATTCCTC 1170
Thr Ala His Thr Ser Ala Trp Asp Phe Phe Ser Gln Gln Ser Thr Ala Leu His Ser Ala Leu Trp Leu Met Ser Gly Asn Gly Ile Pro
GTTCTTTCCGCCACATGAACGGCTACGGAGTCCACAGCTTCCGCTTCGTCGCTGCCAATGGCACTTCCAAGGTGGTGCGAACACCTTGGA 1260
Arg Ser Phe Arg His Met Asn Gly Tyr Gly Val His Ser Phe Arg Phe Val Ala Ala Asn Gly Thr Ser Lys Val Val Arg Thr Pro Trp
AGTCCCAACAGGGTGTTGCCAGTCTGGTGTGGGATGAAGCTCAGGCCGCTGCTGGTAAGAACAGTGACTACCACCGCCAGGATCTGTACA 1350
Lys Ser Gln Gln Gly Val Ala Ser Leu Val Trp Asp Glu Ala Gln Ala Ala Ala Gly Lys Asn Ser Asp Tyr His Arg Gln Asp Leu Tyr
ATGCGATGCCCAATGGCCACTACCCGAAATACGAGGTCAGCCAATCCCTTGATGTCTATCGATAGAGCCTTTTGCTGACAATCCCCTAGG 1440
Asn Ala Met Pro Asn Gly His Tyr Pro Lys Tyr Glu - - - - - - - - - - - - - - - - - - - - - - - - - - - - - - - - - - - - -
TCCAAGCCCAGATCATGGATGAGGCTGACATGCTTCGTTTCGGCTTCGACCTTCTGGATCCCACCAAGTTGGTCCCCGAGGAGGTTGTCC 1530
Leu Gln Ala Gln Ile Met Asp Glu Ala Asp Met Leu Arg Phe Gly Phe Asp Leu Leu Asp Pro Thr Lys Leu Val Pro Glu Glu Val Val
CTTACACTCCTCTCGGAATGATGGAGCTCAATGCCAACCCCACCAACTACTTTGCTGAAGTTGAACAGGCTGGTGTATGTATTCCCCATT 1620
Pro Tyr Thr Pro Leu Gly Met Met Glu Leu Asn Ala Asn Pro Thr Asn Tyr Phe Ala Glu Val Glu Gln Ala Gly - - - - - - - - - - -
CATCAAATGCCAGACATAATCTAACTTCTGCAGTTCCAACCCGGTCACGTCGTTCCTGGCATTGACTTCACCGACGACCCCCTGCTGCAA 1710
- - - - - - - - - - - - - - - - - - - - - - - Phe Gln Pro Gly His Val Val Pro Gly Ile Asp Phe Thr Asp Asp Pro Leu Leu Gln
GGCCGTCTCTTCTCCTACCTCGACACTCAGTTGACCCGTCACGGCGGTCCCAACTTCGAGCAAATCCCCGTCAACCGTCCTCGCAAGCCC 1800
Gly Arg Leu Phe Ser Tyr Leu Asp Thr Gln Leu Thr Arg His Gly Gly Pro Asn Phe Glu Gln Ile Pro Val Asn Arg Pro Arg Lys Pro
GTTCACAACAACAACCGTGACGGCTTCGGCCAGCAGCAGATCCCCACCAACAACTGGGCCTACACCCCCAACAGCATGAGCAACGGTTAC 1890
Val His Asn Asn Asn Arg Asp Gly Phe Gly Gln Gln Gln Ile Pro Thr Asn Asn Trp Ala Tyr Thr Pro Asn Ser Met Ser Asn Gly Tyr
CCCATGCAAGCCAACCAGACCCAGGGTCATGGTTTCTTCACCGCGCCCTACCGCTACGCTTCCGGCCATCTCGTCCGCCAGACCAGCCCG 1980
Pro Met Gln Ala Asn Gln Thr Gln Gly His Gly Phe Phe Thr Ala Pro Tyr Arg Tyr Ala Ser Gly His Leu Val Arg Gln Thr Ser Pro
ACCTTCAATGACCACTGGTCCCAGCCCGCCATGTTCTGGAACTCTCTGATCCCCGCTGAGCAGCAGATGGTTGTCAACGCCATTGTCTTT 2070

FIG. 5B

Thr Phe Asn Asp His Trp Ser Gln Pro Ala Met Phe Trp Asn Ser Leu Ile Pro Ala Glu Gln Gln Met Val Val Asn Ala Ile Val Phe
GAGAACTCCAAGGTTAACAGCCCCCACGTTCGGAAGAACGTTGTCAACCAGCTGAACATGGTCAACAACAACCTCGCCGTCCGTGTCGCT 2160
Glu Asn Ser Lys Val Asn Ser Pro His Val Arg Lys Asn Val Val Asn Gln Leu Asn Met Val Asn Asn Asn Leu Ala Val Arg Val Ala
CGTGGTCTTGGTCTCGATGAGCCCTCCCCCAACCCGACTTACTACACCTCCAACAAGACCTCCAACGTCGGTACCTTCGGCAAGCCCCTC 2250
Arg Gly Leu Gly Leu Asp Glu Pro Ser Pro Asn Pro Thr Tyr Tyr Thr Ser Asn Lys Thr Ser Asn Val Gly Thr Phe Gly Lys Pro Leu
CTCAGCATCGAGGGTCTGCAGGTCGGCTTCCTGGCCTCGAACTCCCACCCCGAATCCATCAAGCAGGGCCAGGCCATGGCCGCGCAGTTC 2340
Leu Ser Ile Glu Gly Leu Gln Val Gly Phe Leu Ala Ser Asn Ser His Pro Glu Ser Ile Lys Gln Gly Gln Ala Met Ala Ala Gln Phe
TCTGCCGCTGGCGTCGACCTGAACATTGTCACCGAGGCCTACGCCGATGGTGTCAACACCACCTACGCCCTGTCTGATGCCATCGACTTT 2430
Ser Ala Ala Gly Val Asp Leu Asn Ile Val Thr Glu Ala Tyr Ala Asp Gly Val Asn Thr Thr Tyr Ala Leu Ser Asp Ala Ile Asp Phe
GACGCCCTCATCATCGCCGATGGTGTGCAGAGCCTCTTCGCCTCCCCCGCTCTCGCTAACCAGATGAACTCTACCGCCACCTCTACTCTC 2520
Asp Ala Leu Ile Ile Ala Asp Gly Val Gln Ser Leu Phe Ala Ser Pro Ala Leu Ala Asn Gln Met Asn Ser Thr Ala Thr Ser Thr Leu
TACCCTCCTGCCAGACCTTTCCAGATCCTGGTCGATTCTTTCAGGTACGGTAAGCCCGTGGCTGCTGTCGGCAGTGGCAGTGTTGCGCTC 2610
Tyr Pro Pro Ala Arg Pro Phe Gln Ile Leu Val Asp Ser Phe Arg Tyr Gly Lys Pro Val Ala Ala Val Gly Ser Gly Ser Val Ala Leu
AAGAACGCTGGTATTGATTCCTCCCGCTCTGGTGTGTACACTGGCTCGAGCGAGACGACGGAGAAGATCGCCAAGGAGGTCTTGGAGGGA 2700
Lys Asn Ala Gly Ile Asp Ser Ser Arg Ser Gly Val Tyr Thr Gly Ser Ser Glu Thr Thr Glu Lys Ile Ala Lys Glu Val Leu Glu Gly
CTCTACACTTTCCGTTTTGTGGACCGGTTTGCGCTGGATGAGTAAGGGTATCACGTTTGTACTTGTACTCACGTTCATCGTTTGTGATGA 2790
Leu Tyr Thr Phe Arg Phe Val Asp Arg Phe Ala Leu Asp Glu
TACATTGATTGATCGATAGATATTTTGTGAGATAGATAGAGTATACTAGAGWGKACATATCTCTACTGATGAGGTGTTGTGCTGCTGCAA 2880
CACATATTTATGAATATATATTCTCTTCTTTGTGAAAGCTAGCCTTCTATATAATCAGCAATGGTTAACTCTTCCAATTCTATAGATACC 2970
AATCACCTAACCCACTCGGAATGACGACAGAAAACATCGACATGTTCGCCCAAGTAAAGCTACTTGAACTTCTACATTTATGCTATGCTG 3060
GAGTCCTCTCATAAGTCCAGAATAAACAAAGAGATCCGATCCTGCTC 3107

| |
|---|
| FIG. 5A |
| FIG. 5B |
| FIG. 5C |

KEY TO FIG. 5

FIG. 5C

Figure 6

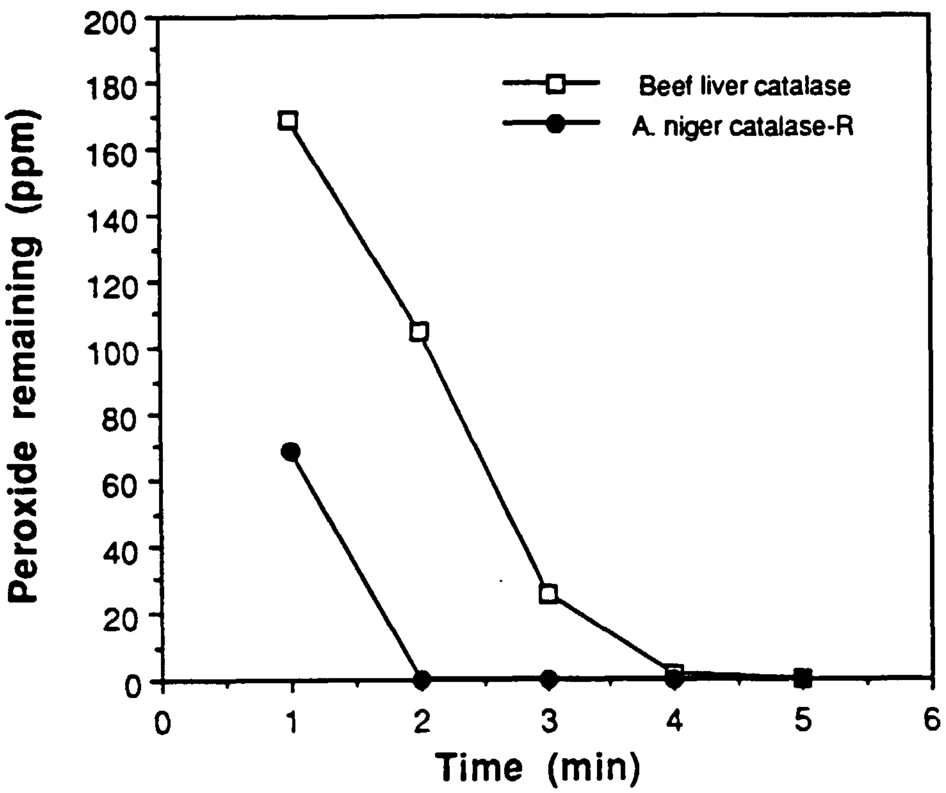
Beef liver catalase
A. niger catalase-R
Peroxide remaining (ppm)
Time (min)
0
20
40
60
80
100
120
140
160
180
200
0
1
2
3
4
5
6